(19) Europäisches Patentamt
European Patent Office
Office européen des brevets

(11) Publication number: **0 011 321**
**A1**

(12) **EUROPEAN PATENT APPLICATION**

(21) Application number: 79200585.2

(51) Int. Cl.³: C 07 D 209/52

(22) Date of filing: 15.10.79

(30) Priority: 27.10.78 GB 4228178

(43) Date of publication of application:
28.05.80 Bulletin 80/11

(84) Designated Contracting States:
AT BE CH DE FR GB IT NL SE

(71) Applicant: SHELL INTERNATIONALE RESEARCH MAATSCHAPPIJ B.V.
Carel van Bylandtlaan 30
NL-2596 HR DEN HAAG(NL)

(72) Inventor: Mason, Ronald Frank
"Kingswood" Westwell
Near Ashford, Kent(GB)

(74) Representative: Keuzenkamp, Abraham et al,
c/o Shell Internationale Research Maatschappij B.V.
P.O. Box 302
NL-2501 CH 's-Gravenhage(NL)

(54) **Pyrrolidine derivatives and process for the preparation of such compounds.**

(57) 3-Azabicyclo[3.1.0]hexane -2,4-dione and substituted analogues thereof can be prepared by reacting a compound of the general formula II or a halide, ester or anhydride thereof:-

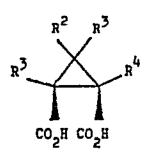

(II)

in which each of $R^1$, $R^2$, $R^3$ and $R^4$ represents a hydrogen atom or an optionally alkoxy-substituted alkyl, alkenyl, aryl, alkaryl or aralkyl group, with ammonia and optionally water, and effecting cyclisation of the resultant intermediate product by heating. Most of the compounds prepared are novel.

EP 0 011 321 A1

Croydon Printing Company Ltd.

"PYRROLIDINE DERIVATIVES AND PROCESS FOR THE PREPARATION OF SUCH COMPOUNDS"

This invention relates to a process for the preparation of certain pyrrolidine derivatives which are useful intermediates in the preparation of biologically active molecules. Most of the compounds are novel.

Certain derivatives of 2-carboxy-3-azabicyclo[3.1.0]hexane have useful plant growth regulating activity, being capable of sterilizing the male anthers of plants. The 3-azabicyclo [3.1.0]hexane ring system is however very difficult to synthesise. A method has now been found for the preparation of certain pyrrolidine derivatives which may be used in the preparation of 2-carboxy-3-azabicyclo[3.1.0]hexane derivatives thereof.

The invention provides a process for the preparation of a compound of the general formula:-

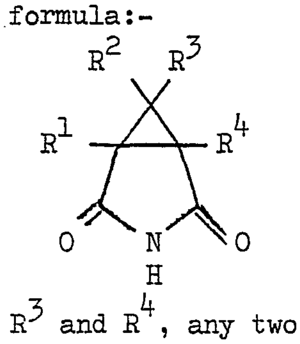

wherein each of $R^1$, $R^2$, $R^3$ and $R^4$, any two or more of which may be the same or different, represents a hydrogen atom or an alkyl, alkenyl, aryl, alkaryl or aralkyl group which may be unsubstituted or substituted by one or more alkoxy groups; or $R^1$ and $R^4$ have the above meaning and $R^2$ and $R^3$ together represent an alkylene group; which comprises reacting a cyclopropane derivative which is a compound of the general formula:

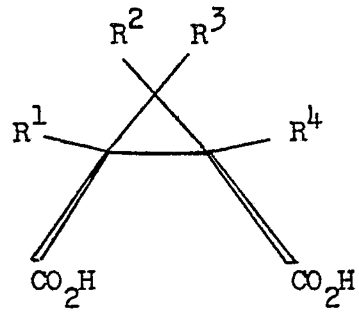

(II)

wherein $R^1$, $R^2$, $R^3$ and $R^4$ have the meanings given for the general formula I, or a mono- or di-acyl halide or a mono- or di-ester or the anhydride thereof, with ammonia and optionally water; and effecting cyclisation by subjecting the resultant product to elevated temperature.

Suitable cyclopropane derivatives to be used as starting materials for the process according to the invention include the free cis-acid of formula II and the anhydride thereof. If an acyl halide is used, this is preferably the di-acyl chloride. If an ester is used, this is preferably a dialkyl ester, for example the dimethyl or diethyl ester. In all cases, a cis-compound must be used, in order that cyclisation may be effected after the treatment with ammonia. The most preferred cyclopropane derivative starting material is the anhydride of the acid of the formula II.

Preferably each of $R^1$, $R^2$, $R^3$ and $R^4$ represents a hydrogen atom or an unsubstituted alkyl or alkenyl group having up to 6 carbon atoms, or an unsubstituted aryl, alkaryl or aralkyl group having up to 10 carbon atoms; or $R^2$ and $R^3$ together form an alkylene group containing up to 6 methylene groups.

More preferably each of $R^1$, $R^2$, $R^3$ and $R^4$ represents a hydrogen atom or an unsubstituted alkyl or alkenyl group having up to 4 carbon atoms, for example a methyl group; or $R^2$ and $R^3$ together form an alkylene group containing 4 or 5 methylene groups.

An especially preferred embodiment of the process according to the invention is one in which 3-aza bicyclo[3.1.0]hexane-2,4-dione of formula

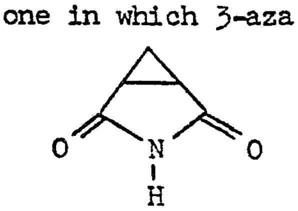

(III)

is prepared.

The cyclopropane derivative starting material may be reacted with gaseous ammonia, with liquid ammonia or with a solution of ammonia, for example an aqueous solution of ammonia. If an aqueous solution is used, this is preferably a concentrated solution, for example .880 ammonia (i.e. a saturated solution). The reaction of the cyclopropane derivative with the aqueous ammonia preferably takes place at a temperature in the range of 10 to 100°C, preferably 15 to 50°C; most preferably, the reaction, which is generally exothermic, is performed without external heating.

Preferably, however, gaseous ammonia is used as reactant. In this case, the reaction is preferably carried out at a temperature in the range 50 to 200°C, more preferably 80 to 150°C, especially 100 to 140°C. The reaction may if desired be conducted under pressure, for example under a pressure of up to 100 atmospheres, but is preferably conducted under atmospheric pressure.

An intermediate product is formed during the process according to the invention, the nature of which depends on the starting materials and the reaction conditions used. If for example the starting material is the free acid of the formula II, or if the acid anhydride is reacted with ammonia and with water, the mono- or di- ammonium salt of the acid of the formula II is formed, depending on the quantity of ammonia used. The salt may if desired be isolated from the reaction mixture. Heating yields the desired compound of the general formula I. Suitably the salt is heated in an inert atmosphere, for example of nitrogen or a noble gas, and preferably to a temperature in the range 150 to 300°C, especially 175 to 275°C.

However, if for example the cyclopropane derivative used as starting material is an acyl halide or an ester, or if gaseous ammonia is reacted with the acid anhydride, the reaction generally proceeds via formation of one of the intermediates

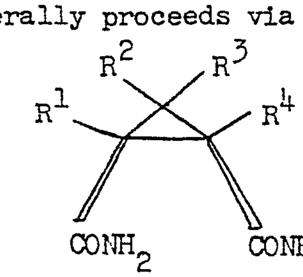 (IV) or 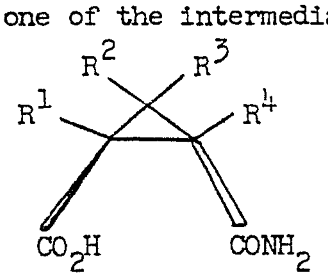 (V)

which generally need not be isolated from the reaction mixture. It is generally preferred to prepare as intermediate a compound of the formula V, as this may be cyclised at a lower temperature than the compound of formula IV. Suitably, the ammonia is bubbled through the reaction mixture which is preferably maintained at a temperature in the range 65-150°C, preferably 100-140°C. When from one to two moles, preferably substantially one mole, of ammonia has been taken up per mole of cyclopropane derivative starting material, the reaction mixture is heated preferably to a temperature in the range 150-300°C, to cyclise the compound of the general formula IV or V. Suitably this heating is carried out under an inert atmosphere. A compound of formula IV is preferably heated to a temperature in the range 200-300°C, more preferably 250-280°C in order to effect cyclisation, while a compound of formula V is preferably heated to a temperature in the range 150 to 250°C, preferably 160 to 180°C.

Most of the compounds of the general formula I are novel. The invention therefore provides a compound of the general formula I as defined above, with the provisos that, if $R^1$ represents an ethyl or p-chlorophenyl group, then at least one of $R^2$, $R^3$ and $R^4$ is other than a hydrogen atom, and if $R^1$ represents a phenyl group and $R^2$ represents a hydrogen atom or a methyl group, then at least one of $R^3$ and $R^4$ is other than a hydrogen atom.

The following Examples illustrate the invention. In each Example, the identity of the product was confirmed using elemental analysis and NMR and infra red spectral analysis.

Example 1

Cyclopropane-1,2-dicarboxylic anhydride was mixed with .880 ammonia solution in the molar ratio anhydride:$NH_3$ approximately 1:2. A very exothermic reaction ensued, and a homogeneous solution was formed. The solvent was stripped on a rotary evaporator, and the resulting glass was allowed to crystallise.

The resulting solid diammonium cyclopropane-1,2-dicarboxylate was heated at 200°C for 10 minutes and then at 260-275°C for a further 20 minutes. During the heating, water was distilled off and ammonia was evolved.

The cooled melt was triturated with ethanol, and the resulting solid was filtered and sucked dry. The crude solid sublimed at 0.1 mm pressure and 100°C, and the resulting white solid was re-crystallised from ethanol to give colourless crystals of 3-aza-bicyclo[3.1.0]hexane-2,4-dione, M.Pt. 97-99°C. The IR spectrum showed absorption peaks at 3140 $cm^{-1}$, 1755 $cm^{-1}$ and 1675 $cm^{-1}$. Elemental analysis was as follows:

| | | | |
|---|---|---|---|
| Calculated for $C_5H_5O_2N$ | C54.3; | H4.6; | N12.6 |
| Found | C54.1; | H4.5; | N12.6 |

Example 2

112g of 1,2-cyclopropanedicarboxylic anhydride were weighed into a 250 ml flask fitted with a gas inlet pipe, a condensor and a thermometer. The anhydride was warmed to 86°C and ammonia was bubbled into the flask, which was cooled by a stream of air. 18g of ammonia were added over the course of an hour, during which the temperature rose to 148°C and then fell to 112°C.

A stream of nitrogen was then flushed through the flask, which was warmed to 170°C to dehydrate the reaction mixture. The reaction mixture was then cooled to 100°C and poured into 250 ml isopropyl alcohol cooled to 0°C on a carbon dioxide/isopropyl alcohol bath. The resulting solid was filtered off, washed with cold petroleum (B.Pt. 60-70°C), and air-dried overnight. 79.3g of 3-aza-bicyclo[3.1.0]hexane-2,4-dione as beige crystals, M.Pt. 94-96°C, were obtained.

Example 3

15g of cis 1,2-diethoxycarbonylcyclopropane was stirred with 150 ml of .880 ammonia for 3 days. The resulting mixture, which was homogeneous except for a few oily drops, was stripped on a rotary evaporator to give 11.0g of an orange-yellow, sticky solid. After recrystallisation from demineralised water, 4.5g of cis 1,2-diaminocarbonylcyclopropane were obtained as crystals, M.Pt. 243-245°C.

1g of this product was heated, under a nitrogen blanket, at 265-270°C. Vigorous effervescence caused by liberation of ammonia ensued, and ceased after 15-20 minutes. Trituration of the resulting cooled melt with ethanol gave a brownish solid which was recrystallised from ethanol. Crystals of 3-azabicyclo[3.1.0]hexane-2,4-dione, M.Pt. 97-99°C, were obtained.

CLAIMS

1. A process for the preparation of a compound of the general formula:-

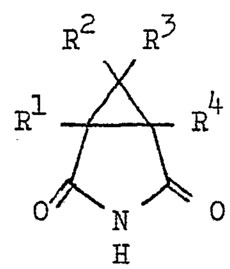

(I)

wherein each of $R^1$, $R^2$, $R^3$ and $R^4$, any two or more of which may be the same or different, represents a hydrogen atom or an alkyl, alkenyl, aryl, alkaryl or aralkyl group which may be unsubstituted or substituted by one or more alkoxy groups; or $R^1$ and $R^4$ have the above meanings and $R^2$ and $R^3$ together represent an alkylene group; characterized in that a cyclopropane derivative of the general formula:-

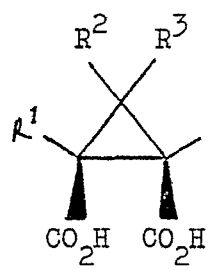

(II)

wherein $R^1$, $R^2$, $R^3$ and $R^4$ have the meanings given for the general formula I, or a mono- or di-acyl halide or a mono- or di-ester or the anhydride thereof, is reacted with ammonia and optionally water; and cyclisation is effected by subjecting the resultant intermediate produced to elevated temperature.

2. A process as claimed in claim 1, characterized in that the cyclopropane derivative starting material is the free cis acid of formula II or the anhydride thereof.

3. A process as claimed in either claim 1 or claim 2, characterized in that a starting material is used in which each of $R^1$, $R^2$, $R^3$ and $R^4$ represents a hydrogen atom.

4. A process as claimed in any one of claims 1 to 3, characterized in that the cyclopropane derivative is reacted with aqueous ammonia at a temperature in the range of from 10 to 100°C.

5. A process as claimed in any one of claims 1 to 3, characterized in that the cyclopropane derivative is reacted with gaseous ammonia at a temperature in the range of from 50 to 200°C.

6. A process as claimed in any one of claims 1 to 5, characterized in that cyclisation is effected by heating the resultant intermediate product to a temperature in the range of from 150 to 300°C.

7. A compound of the general formula I given in claim 1, in which $R^1$, $R^2$, $R^3$ and $R^4$ have the meanings given in claim 1, with the provisos that, if $R^1$ represents an ethyl or p-chlorophenyl group, then at least one of $R^2$, $R^3$ and $R^4$ is other than a hydrogen atom, and if $R^1$ represents a phenyl group and $R^2$ represents a hydrogen atom or a methyl group, then at least one of $R^3$ and $R^4$ is other than a hydrogen atom.

8. 3-Azabicyclo[3.1.0]hexane-2,4-dione.

European Patent Office

# EUROPEAN SEARCH REPORT

0011321

Application number

EP 79 20 0685

## DOCUMENTS CONSIDERED TO BE RELEVANT

| Category | Citation of document with indication, where appropriate, of relevant passages | Relevant to claim |
|---|---|---|
| X | US - A - 3 745 170 (A. FUJINAMI)<br>* Column 1, column 37, examples 1-13; column 13, table 7 *<br>-- | 1-6 |
| X | FR - A - 2 327 989 (SUMITOMO)<br>* Page 19, claim 6 *<br>---- | 1-6 |

CLASSIFICATION OF THE APPLICATION (Int. Cl. 3)

C 07 D 209/52

TECHNICAL FIELDS SEARCHED (Int.Cl. 3)

C 07 D 209/52

CATEGORY OF CITED DOCUMENTS

- X: particularly relevant
- A: technological background
- O: non-written disclosure
- P: intermediate document
- T: theory or principle underlying the invention
- E: conflicting application
- D: document cited in the application
- L: citation for other reasons

&: member of the same patent family, corresponding document

The present search report has been drawn up for all claims

| Place of search | Date of completion of the search | Examiner |
|---|---|---|
| The Hague | 13-02-1980 | MAISONNEUVE |

EPO Form 1503.1 06.78